Europäisches Patentamt

European Patent Office

Office européen des brevets

(11) Veröffentlichungsnummer: **0 444 673 A2**

## (12) EUROPÄISCHE PATENTANMELDUNG

(21) Anmeldenummer: 91103017.9

(22) Anmeldetag: 28.02.91

(51) Int. Cl.⁵: **A61K 35/64**, A61K 9/66, D06M 16/00, D06M 13/224

(30) Priorität: 02.03.90 DE 4006481
09.01.91 DE 4100401

(43) Veröffentlichungstag der Anmeldung:
**04.09.91 Patentblatt 91/36**

(84) Benannte Vertragsstaaten:
**AT BE CH DE DK ES FR GB GR IT LI LU NL SE**

(71) Anmelder: **Ludwig, Wolfgang, Dr.**
**Silcherstrasse, 21**
**W-7240 Horb 1(DE)**

(72) Erfinder: **Ludwig, Wolfgang, Dr.**
**Silcherstrasse, 21**
**W-7240 Horb 1(DE)**

(74) Vertreter: **Ott, Elmar, Dipl.-Ing.·**
**Kappelstrasse 8**
**W-7240 Horb 1(DE)**

(54) **Emulsion aus Bienenprodukten.**

(57) Eine Emulsion aus Bienenprodukten und damit getränkte Materialien werden in ihrer kristallinflüssigen Struktur so modifiziert, daß sie ein gegenphasiges Spektrum zu geopathischen Störsignalen aufweisen und zur Kompensation geopathischer Störsignale verwendet werden können.

Diese Strukturmodifikation wird mit Hilfe konventioneller Breitbandverstärker (6) mit Invertierstufe (7) durchgeführt, wobei zur Phasensynchronisation der Haupteigenwert der natürlichen Schumannwellen benutzt wird. Die Schwingungs-Information von geopathischen Plätzen wird den vier Naturbereichen Mineral (5), Pflanze, Tier, Mensch mit konventionellen Radiometern (4) und Signalanalysatoren entnommen. Weitere Zusatzinformationen und magnetische Felder können die Wirkung verbessern.

Fig. 2

EP 0 444 673 A2

Die Erfindung betrifft eine Emulsion aus Substanzen, die von Bienen gesammelt bzw. produziert werden und die getränkt in Stoffgeweben für gesundheitliche Zwecke, vorzugsweise in Sitz- und Liegemöbeln als Einlage verwendet wird.

In der Deutschen Patentschrift 35 33 864 ist ein Duftgemisch aus Bienenprodukten (Bienenwachs, Propolis usw.) beschrieben, das als Schmelze oder mittels organischer Lösungsmittel verarbeitet wird. Mit diesem Duftgemisch werden Stoffgewebe, Sitz- und Liegemöbel versehen, um durch seine flüchtigen Bestandteile (insbesondere von Propolis) gesundheitsfördernde Eigenschaften zu erhalten.

Die gesundheitsfördernde Wirkung von Propolis ist bekannt. Ebenso ist bekannt, daß Propolis eine ganze Reihe leichtflüchtiger Bestandteile enthält, die über die Haut und Atmungsorgane von Menschen aufgenommen werden und dadurch ihre Wirkung im Organismus entfalten können (Aroma-Therapie).

Nachteil der bisherigen Verarbeitung war die Notwendigkeit, Bienenwachs bei höheren Temperaturen zu schmelzen, wobei die flüchtigen Bestandteile des zugesetzten Propolis schneller verdunsten als erwünscht, d.h. man verliert während des Schmelzens und Mischens bereits wertvolle Bestandteile von Propolis. Die Verarbeitung mit organischen Lösungsmitteln hat den Nachteil, daß diese ausnahmslos feuergefährlich sind und explosionsgeschützte Maschinen für das Auftragen auf Stoffgewebe usw. erforderlich machen.

Die vorliegende Erfindung geht aus von dem DE-Patent 35 33 864 und stellt sich die Aufgabe, die geschilderten Nachteile zu umgehen. Erfindungsgemäß wird vorgeschlagen, anstelle einer Schmelze oder der Verwendung von organischen Lösungsmitteln eine Emulsion in Wasser zu verwenden. Die Erfahrung hat gezeigt, daß vor allem Bienenwachs und Propolis wesentliche Bestandteile sind, so daß weitere Komponenten auch wegfallen können. Damit vereinfacht sich die Herstellung. Insgesamt kommen folgende Vorteile zum tragen:

- problemlose und schonende Verarbeitung auf Wasserbasis ohne Geruchsbelästigung und umweltfreundlich.
- einfache und preiswerte Herstellung
- längere Wirkungsdauer, da die flüchtigen Bestandteile aus dem Emulgat langsamer abgegeben werden als bei Verwendung von organischen Lösungsmitteln.

Die Anwendung kann die gleiche sein, wie in dem DE-Patent 35 33 864 beschrieben.

In der deutschen Patentanmeldung P 40 06 481.6-41 vom 27.01.1990 ist eine wässrige Emulsion aus Bienenwachs und Propolis beschrieben, die zum Tränken von Materialien geeignet ist und gesundheitsfördernde Wirkung haben kann.

Nachteil war bisher, daß die getränkten Materialien nur bei schwachen tellurischen Umwelteinflüssen wirkten und andernfalls größerer technischer Aufwand erforderlich wurde, wie z.B. magnetische Abschirmung.

Die Erfindung stellt sich weiterhin die Aufgabe, diesen Nachteil zu beseitigen. Dazu wurde die genannte Emulsion weiterentwickelt, nämlich mit einer speziellen Strukturmodifikation versehen, die eine Verbesserung der Wirkung zum Ziel hat und somit einen größeren technischen Aufwand erübrigt.

Es ist heute nicht mehr zu leugnen, daß es für den Menschen ungünstige Standorte gibt, bei denen terrestrische Umwelteinflüsse eine Rolle spielen (1). Aus Untersuchungen des Erfinders geht hervor, daß hierbei periodische Schwankungen elektromagnetischer Felder eine Rolle spielen, die u.a. in ihrer Phasenlage durch den Haupteigenwert der Schumannwellen (2) synchronisiert werden. Bekannt ist, daß polare Substanzen Informationen speichern können (3). Dies ist am längsten erforscht bei magnetischen Dipolen im Eisen aber ebenso lange bekannt bei elektrischen Dipolen in kristallinen Flüssigkeiten wie Wasser, Alkoholen, Fetten, Harzen u.a.

Weiter ist bekannt, daß gesunde höhere Organismen in der Lage sind, langanhaltende äußere Störeinflüsse kybernetisch zu kompensieren (2). Der Organismus erzeugt dabei u.a. ein Gegenfeld bzw. eine Gegeninformation zum Störsignal. Ein analoges Verfahren wird seit vielen Jahren in der Medizin angewandt (4). Bekannt ist auch, daß Pflanzen, die auf Störplätzen wachsen, geopathische Informationen speichern, die in der Medizin angewandt werden kann (5).

Schließlich ist es eine langjährige Erfahrung, daß junge Menschen die sich vegetarisch ernähren und regelmäßig Yogaübungen ausführen, ein besonders harmonisches Schwingungsspektrum aufweisen, das sich radiometrisch bzw. mittels Signalanalysatoren messen läßt (6).

Auf der Basis dieser bekannten Tatsache wird erfindungsgemäß vorgeschlagen, die in der deutschen Patentanmeldung P 40 06 481.6-41 genannte Emulsion auf folgende Weise strukturmodifiziert zu behandeln, was sich bereits im Doppelblindtest praktisch bewährt hat.

1. Von den vier Naturbereichen Mineral, Pflanze, Tier, Mensch wird jeweils ein Schwingungs-Spektrum abgegriffen und zwischengespeichert:

a) von standortgemäßen Mineralien an stark gestörten geopathischen Plätzen, erkenntlich an erhöhter Gamma-Bodenstrahlung (wie z.B. von Prof. Dr. Betz, Univ. München in der Garchinger Heide festgestellt).

b) von standortgemäßen Pflanzen auf stark gestörten geopathischen Plätzen.

c) von "strahlensuchenden" Tieren wie z.B. Katzen, die sich am liebsten auf geopathischen Plätzen aufhalten.

d) von Jugendlichen, die sich vegetarisch ernähren und regelmäßig Yogaübungen ausführen und sich freiwillig einige Stunden lang auf einem stark geopathisch gestörten Platz aufhalten.

Auf diese Weise ist garantiert, daß möglichst viele der unterschiedlichen Umwelteinflüsse erfaßt werden.

2. Die von diesen vier Naturbereichen abgegebenen elektromagnetischen Felder (radiometrisch und spektroskopisch abgreifbar und meßbar, 7) werden einzeln über einen elektronischen Verstärker mit Leistungsausgang auf polares Material, z.B. ferromagnetisches Material zwischengespeichert, wobei die Struktur gleichgerichteter Bezirke (im Falle ferromagnetischen Materials als WEISSsche Bezirke, im Falle elektrischer Dipole Cluster) und der dazwischen liegenden Knotenflächen (Blochwände, 8, bzw. kinks) durch erzwungene Platzwechsel charakteristisch modifiziert werden.

3. Durch eine Invertierstufe, die von den Schumannwellen synchronisiert wird, wird eine breitbandige Phasenumkehr erreicht, so daß ein Gegenspektrum zu dem tellurischen Schadspektrum entsteht (4).

Am Leistungsausgang des Verstärkers ist eine Spule angeschlossen, in die das polare Material zur Speicherung eingebracht ist.

4. Die Zwischenspeicherung wird spektroskopisch für jedes der vier polaren Materialien (den vier Naturbereichen zugeordnet) erfaßt und das Mischungsverhältnis optimiert. Hierzu wird ein Summationsverstärker verwendet, der vier Spuleneingänge und einen Spulenausgang hat. In der Ausgangsspule befindet sich ein fünftes polares Material, in dem die Summe der vier Informatioen gesammelt wird. Die Optimierung erfolgt durch physiologische Tests an mehreren Versuchspersonen mittels bekannter Verfahren wie Kinesiologie (10) Georhythmogramm (11), Flimmerverschmelzungstest (2) und Thermoregulationsdiagnose (12).

Das Ergebnis kann erfindungsgemäß noch wie folgt verbessert werden: Weitere polare Materialien werden mit Substanzen strukturmodifiziert, die bekanntermaßen für den menschlichen Organismus nützlich sind. Hierzu gehören Multiplasane, Prolixane, Regenaplexe und homöopathische Ausleitungsmittel wie Solidago und Schwermetall-Hochpotenzen. Diese Informationen können über zusätzliche Eingänge des erwähnten Summenverstärkers eingespeist und wie oben erwähnt optimiert werden.

Das polare Summenmaterial, in dem sämtliche genannte Informationen gespeichert wurden, weist nun bestimmte Solitonenspektren auf (9), die durch Schwingungen der Knotenflächen definiert sind (im Falle elektrischer Dipole kinks, im Falle magnetischer Dipole Blochwände).

Nach einer möglichen nochmaligen Optimierung auf Grund eines physiologischen Testergebnisses, wie oben erwähnt, wird der letzte Schritt der Strukturmodifikation der Emulsion auf Bienenwachs und Propolis durchgeführt. Erfindungsgemäß wird hierzu das polare Summenmaterial in eine Eingangsspule des Summenverstärkers gegeben (die anderen Eingänge bleiben hierbei unbenutzt) und in die Ausgangsspule wird die Emulsion gegeben. Nach Tränken von Materialien, z.B. Stoffbändern, können diese zur Verstärkung der Wirkung nochmals ebenso nachstrukturiert werden, wie oben beschrieben. Die erforderliche Zeit beträgt jeweils mehrere Stunden und kann durch physiologische Zwischentests an Versuchspersonen kontrolliert werden, wie oben beschrieben.

Bei der Optimierung aller dieser geschilderten Verfahren hat sich im Laufe der langjährigen Entwicklung herausgestellt, daß auch die Jahreszeit, die Sonnenfleckentätigkeit und die Schwankungen des Erdmagnetfelds beachtet werden müssen, um die bestmögliche Optimierung zu erhalten. Ist dieser gesamte Prozeß einmal erfolgreich durchlaufen und optimiert, kann das polare Summenmaterial immer wieder verwendet werden, so daß der aufwendige Vorgang nur in der Anfangsphase (bei der Zwischenspeicherung) notwendig ist.

Die physiologische Wirkung kann erfindungsgemäß weiter verbessert werden, wenn die erfindungsgemäßen Stoffbänder mit Magnetstreifen unterlegt werden, so daß das Magnetfeld die Information in den Raum transportiert.

Mit der erfindungsgemäßen Anordnung lassen sich terrestrische Umwelteinflüsse weitgehend kompensieren. Die Erfahrung hat gezeigt, daß kranke Tiere in einem gestörten Stall nach Auslegen der erfindungsgemäßen Stoffbänder wieder gesund werden, daß Kleinstkinder, die nachts nicht schliefen, ständig schrieen und sich im Bett in einer Ecke zusammenrollten, nach Einlegen der erfindungsgemäßen Stoffbänder normal schliefen. Ein Wirkungsverlust ist in der Beobachtungszeit von sechs Jahren nicht aufgetreten. Das Störspektrum wird damit natürlich nicht beseitigt oder gar abgeschirmt, aber es wird so überlagert, daß es den Organismus nicht mehr stört (d.h. unterhalb der Ansprechschwelle bleibt).

Vorteile der Erfindung sind die mögliche Optimierung eines anfangs zwar aufwendigen, später aber - nach Erstellung des polaren Summenmaterials - technisch sehr einfachen Verfahrens, der Wegfall aufwendiger Abschirmmaßnahmen an geopathisch gestören Plätzen oder deren Meidung und damit größerer Umzugsnotwendigkeiten sowie die problemlose Anwendung ohne großen technischen Aufwand, wie er bisher notwendig war.

Gegenstände der Erfindung sind die Zwischenspeicherung von tellurischen Störinformationen aus den vier Naturbereichen Mineral, Pflanze, Tier, Mensch in polaren Materialien; die Synchronisation der Phasenlage zum Haupteigenwert der Schumannwellen; die Invertierung dieser Signale, die Summierung der vier Signalspektren und weiterer nützlicher Signale aus der Naturmedizin; die Optimierung der Summation anhand von physiologischen Zwischentests und die Übertragung der Summeninformation auf eine wässrige Emulsion von Bienenwachs und Propolis; die nochmalige Strukturmodifikation des Endprodukts sowie die Unterlegung des Endprodukts mit Magnetstreifen. Weiter ist Gegenstand der Erfindung die Anwendung der strukturmodifizierten Materialien in Bett- und Sitzmöbeln sowie Teppichen.

Objektiver Beweis der Wirksamkeit der erfindungsgemäßen Materialien:

Der Forstoberrat Siegfried Otto hat auf einer starken Störzone, über der Margeritten und andere Pflanzen nicht wuchsen, die erfindungsgemäßen Stoffbänder 30 cm tief eingegraben. Hiernach gediehen alle Pfanzen darüber ausgezeichnet.

Die Erfindung wird anhand eines in der Zeichnung dargestellten Ausführungsbeispiels näher erläutert.

Es zeigen:

Figur 1 eine Auflage für Matratzen oder dergleichen mit eingelegten Bändern, auf denen die erfindungsgemäße Emulsion aufgetragen ist,
Figur 2 eine Radiometeranordnung zur Beeinflußung eines polaren Materials.

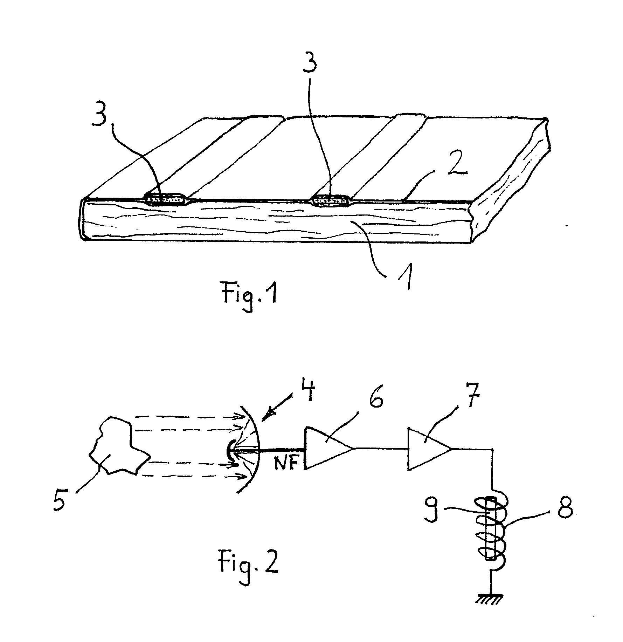

Figur 1 zeigt im Querschnitt eine Auflage für Matratzen, die innen ein Baumwollgewebe 1 hat, das von einem äußeren Überzug 2 umschlossen ist. Zwischen das Baumwollgewebe 1 und den Überzug 2, der ebenfalls aus Baumwolle gefertigt sein kann, sind in Abständen quer verlaufende Bänder 3 eingelegt, die mit einer erfindungsgemäßen Emulsion getränkt sind.

Figur 2 zeigt eine Anordnung mit einem Radiometer 4, welches die Infrarotstrahlung eines Minerals 5 empfängt. Die Modulation dieser Infrarotstrahlung wird über zwei Verstärker 6, 7 verstärkt. Der Ausgang des Verstärkers 7 speist eine Magnetisierungsspule 8, die ein ferromagnetisches Material 9 mit der Niederfrequenz NF aus der empfangenen Infrarotstrahlung magnetisiert.

Das ferromagnetische Material 9 bildet somit einen strukturmodifizierten Zwischenspeicher, mit dem ein weiteres polares Material modifiziert werden kann. Dabei kann dann das ferromagnetische Material 9 anstelle des Minerals 5 verwendet werden.

Es wird noch angemerkt, daß der Versärkter 6 als Breitbandverstärker und der Verstärker 7 als Invertierstufe ausgebildet sein kann.

In Betracht gezogene Druckschriften:

(1) O. Bergsmann: Risikofaktor Standort. Facultas Universitätsverlag Wien, 1990.
(2) H. Wolf: Harmonische Schwingungen. Verlag Natürlich und Gesund, Stuttgart, 1990.
(3) Deutsche Offenlegungsschrift DE 36 12 315 A1
(4) F. Morell: Die MORA-Therapie. Haug-Verlag, Heidelberg, 2. Auflage 1990.
(5) E. Hartmann: Über Konstruktion und Reaktionstypen. Forschungskreis für Geobiologie 1986.
(6) Bigu del Blanco: Some special applications of microwave radiometry of biological systems. Proc. EMC, Montreux 2 nd Symposium, 469-475, 1977.
(7) ECOLOG '88: Umweltzentrum für ökologische Strukturforschung Schloß Türnich, 1988.
(8) G. Heber: Einführung in die Theorie des Magnetismus. Wissenschaftliche Buchgesellschaft, Darmstadt, 1983.
(9) G. Eilenberger: Solitons, Springer-Verlag, 1983.
(10) J. Diamond: Der Körper lügt nicht. Verlag für Kinesiologie, Freiburg i.Br.
(11) E. Hartmann: Krankheit als Standortproblem. 5. Auflage, Haug-Verlag, Heidelberg, 1990.
(12) F. Schmid: Biologische Medizin. Aurelia-Verlag, Baden-Baden, 1990.

**Patentansprüche**

1. Emulsion aus Bienenprodukten, **dadurch gekennzeichnet,** daß Bienenwachs als Grundsubstanz mit folgenden weiteren Substanzen einzeln oder im Gemisch versetzt wird: Propolis, Bienengift, Bienenhonig, Gelee Royal und Blütenpollen, und daß das Gemisch im Wasser emulgiert wird.

2. Emulsion nach Anspruch 1, **dadurch gekennzeichnet,** daß die Emulsion auf folgende Weise strukturmodifiziert wird: Von den vier Naturbereichen Mineral, Pflanze, Tier, Mensch werden standortgemäße Exemplare auf geopathischen Plätzen bzw. freiwillig auf solchen Plätzen postierte Personen dazu benutzt, ihr Schwingungsspektrum durch bekannte Sensoren wie Radiometer und Signalanalysatoren auffangen zu lassen und über einen konventionellen Breitbandverstärker mit mindestens einer Spule am Ausgang auf polares Material wie z.B. ferromagnetisches Material im Inneren der Spule aufprägen zu lassen, wobei vier strukturmodifizierte Zwischenspeicher entstehen, die über einen Breitband-Summierverstärker mit mindestens vier Spuleneingängen und mindestens einem Spulenausgang auf ein weiteres polares Material im Spulenausgang des Breitband-Summierverstärkers übertragen werden, wobei erzwungene Platzwechselvorgänge die Struktur des polaren Materials modifizieren und daß dieses weitere polare Material im Spuleneingang eines konventionellen Breitbandverstärkers die summierte Information der vier Naturbereiche über mindestens eine Ausgangsspule auf die Emulsion von Bienenprodukten aufgeprägt wird, wobei die elektrische Dipole der Emulsion ihre kristallinflüssige Struktur ändern.

3. Emulsion nach Anspruch 1, **dadurch gekennzeichnet,** daß der konventionelle Breitbandverstärker eine Invertierstufe enthält oder diese ihm nachgeschaltet ist.

4. Emulsion nach einem der Ansprüche 2 oder 3, **dadurch gekennzeichnet,** daß die Invertierstufe einen Synchronisations-Eingang besitzt.

5. Emulsion nach einem der Ansprüche 2 bis 4, **dadurch gekennzeichnet,** daß in den Synchronisationseingang der Invertierstufe die Grundwelle der natürlichen Schumannwellen eingespeist wird.

6. Emulsion nach einem der Ansprüche 2 bis 5, **dadurch gekennzeichnet,** daß der genannte konventionelle Breitband-Summierverstärker mindestens einen fünften Eingang zur Einspeisung weiterer Informationen enthält.

7. Emulsion von Bienenprodukten nach Anspruch 6, **dadurch gekennzeichnet,** daß an den fünften Eingang und gegebenenfalls an weiteren Eingängen des genannten konventionellen Breitband-Summierverstärkers die Schwingungsspektren von Multiplasanen, Prolixanen, Regenaplexen und bekannnten homöopathischen Ausleitungsmitteln gegeben werden.

8. Emulsion nach Anspruch 7, **dadurch gekennzeichnet,** daß das Verhältnis der Signale aus den vier Naturbereichen und weiterer Zusatzinformationen dadurch optimiert wird, daß physiologische Zwischentests eingeschoben werden und deren Ergebnisse zur Optimierung herangezogen werden.

9. Emulsion nach einem der vorhergehenden Ansprüche, **dadurch gekennzeichnet,** daß mit einer strukturmodifizierten Emulsion saugfähige Materialien getränkt werden.

10. Emulsion nach Anspruch 9, **dadurch gekennzeichnet,** daß das getränkte Material nochmals einer Strukturmodifikation nach den vorhergehenden Ansprüchen unterzogen wird.

11. Emulsion von Bienenprodukten nach einem der vorhergehenden Ansprüche, **dadurch gekennzeichnet,** daß das getränkte Material mit magnetischem Material unterlegt wird, so daß das Magnetfeld die Informationen übernimmt, d.h. damit moduliert wird und weiter in den Raum transportiert als ohne diese Unterlegung.

12. Anwendung der strukturmodifizierten Materialien nach den vorhergehenden Ansprüchen in Bett-, Sitzmöbeln und Teppichen.

13. Verwendung der Emulsion nach einem der vorhergehenden Ansprüche zum Tränken von Geweben und Holz.

14. Verwendung der Emulsion nach Anspruch 13, **dadurch gekennzeichnet,** daß die Emulsion auf ein Trägermaterial aus Stoffgewebe aufgetragen wird.

15. Verwendung der Emulsion nach Ansprüchen 13 oder 14, **dadurch gekennzeichnet,** daß das Trägermaterial Bandform hat.

16. Verwendung der Emulsion nach Ansprüchen 13 bis 15, **dadurch gekennzeichnet,** daß die Emulsion bzw. das Trägermaterial in Decken, Unterbetten und Kopfkissen eingebracht wird.

Fig.1

NF

Fig. 2